# EUROPEAN PATENT APPLICATION

(11) **EP 4 389 873 A1**
(43) Date of publication of application: **26.06.2024**
(21) Application number: 22858487.6
(22) Date of filing: 17.08.2022
(51) Int. Cl.: C12N 1/00, C12M 1/00, C12M 1/16

(54) **CULTURE METHOD AND CULTURE DEVICE**

(30) Priority: 18.08.2021 JP 2021133193
(71) Applicant: AISIN CORPORATION, Kariya-shi, Aichi 448-8650 (JP)
(72) Inventor: TABATA Yuki, Kariya-shi, Aichi 448-8650 (JP); OTSUBO Isao, Kariya-shi, Aichi 448-8650 (JP)
(74) Representative: Cabinet Beau de Loménie
(86) International application number: PCT/JP2022/031054
(87) International publication number: WO 2023/022169

(57) **Abstract**

A culture method includes: (a) a step of providing a culture medium; (b) a step of supplying fine water from a fine water generation unit to the culture medium, the fine water generation unit being configured to change between a water adsorbing state in which an electrically conductive polymer membrane adsorbs water in air as a temperature decreases and a water releasing state in which the water adsorbed by the electrically conductive polymer membrane is released as fine water as the temperature increases; and (c) a step of inoculating the culture medium supplied with the fine water with a material to be cultured and culturing the material.

## Description

### TECHNICAL FIELD

The present disclosure relates to culture methods and culture devices.

### BACKGROUND ART

Conventionally, microorganisms such as mold, yeast, and bacteria are cultured by inoculating (seeding) a culture medium with them as a material to be cultured. For example, Patent Document 1 describes cultivating (culturing) shiitake fungi while maintaining the humidity in a cultivation chamber at a predetermined value or higher using a device that humidifies intake air and blows it into the cultivation chamber. Patent Document 2 describes inoculating cheese with red koji mold and culturing the red koji mold at a predetermined humidity and a predetermined temperature to cover the surface of the cheese with the red koji mold.

### Related Art Documents

### Patent Documents

Patent Document 1: Japanese Unexamined Patent Application Publication No. 2005-137273 (JP 2005-137273 A)
Patent Document 2: Japanese Patent No. 2871882 (JP 2871882 B)

### SUMMARY OF THE INVENTION

### Problem to be Solved by the Invention

As mentioned above, in order to culture a material to be cultured, it is important to control a culture environment by appropriately controlling humidity and supplying sufficient water. Water is supplied to the material to be cultured not only from air but also from the culture medium. Therefore, there is still room for improvement in order to supply water more appropriately.

It is a primary object of the present disclosure to facilitate culturing by supplying water more appropriately.

### Means for Solving the Problem

The present disclosure took the following measures to achieve the above primary objective.

A first culture method of the present disclosure includes:
(a) providing a culture medium;
(b) a step of supplying fine water from a fine water generation unit to the culture medium, the fine water generation unit being configured to change between a water adsorbing state in which an electrically conductive polymer membrane adsorbs water in air as a temperature decreases and a water releasing state in which the water adsorbed by the electrically conductive polymer membrane is released as fine water as the temperature increases; and
(c) a step of inoculating the culture medium supplied with the fine water with a material to be cultured and culturing the material.

In the first culture method of the present disclosure, after the fine water is supplied to the culture medium from the fine water generation unit that changes between the water adsorbing state and the water releasing state, the culture medium supplied with the fine water is inoculated with the material be cultured, and the material is cultured. Culturing is thus started after a sufficient amount of fine water is supplied to the culture medium. This can facilitate culturing as water is more appropriately supplied to the material to be cultured from the beginning of the culturing.

A second culture method of the present disclosure includes:
(a) providing a culture medium;
(b) supplying fine water with a particle size of 50 nanometers or less to the culture medium; and
(c) a step of inoculating the culture medium supplied with the fine water with a material to be cultured and culturing the material.

In the second culture method of the present disclosure, after the fine water of 50 nanometers or less is supplied to the culture medium, the culture medium supplied with the fine water is inoculated with the material be cultured, and the material is cultured. Culturing is thus started after the fine water with a size that is easily absorbed by the culture medium is supplied to the culture medium. This can facilitate culturing as water is more appropriately supplied to the material to be cultured from the beginning of the culturing.

In the first or second culture method of the present disclosure, in the (b), the fine water may also be supplied to a storage container containing the culture medium while reducing entry of outside air other than air having passed through the fine water generation unit. The culture medium is therefore less likely to be dried by the entry of the outside air, and the storage container can be filled with air containing the fine water. Therefore, supply of fine water to the culture medium can be facilitated.

In the first or second culture method of the present disclosure, in the (c), the storage container containing the culture medium may also be left to stand in a closed state for a predetermined culture period without supplying the fine water. As a sufficient amount of fine water is supplied in advance to the culture medium, culturing can be facilitated by merely letting the culture medium stand after inoculating the culture medium with the material to be cultured. The (c) may be performed at a location away from a location where the (b) is performed. The (c) may further include a step of, after the (b), moving the culture medium to the location away from the location where the (b) is performed. The storage container can thus be left to stand at a location suitable for culturing, and culturing can be facilitated.

A culture device of the present disclosure is a culture device that inoculates a culture medium with a material to be cultured and cultures the material. The culture device includes: an air passage communicating with a storage container; an air blow unit disposed in the air passage; a fine water generation unit that is disposed in the air passage and that changes between a water adsorbing state in which an electrically conductive polymer membrane adsorbs water in air as a temperature decreases and a water releasing state in which the water adsorbed by the electrically conductive polymer membrane is released as fine water as the temperature increases; and a control unit that performs, with the culture medium before being inoculated with the material to be cultured being placed in the storage container, a water adsorption control in which the fine water generation unit is switched to the water adsorbing state and a water release control in which the fine water generation unit is switched to the water releasing state, and controls the fine water generation unit and the air blow unit in such a manner that the fine water is supplied to the culture medium in the storage container by blowing air.

In the culture device of the present disclosure, the water adsorption control in which the fine water generation unit is switched to the water adsorbing state and the water release control in which the fine water generation unit is switched to the water releasing state are performed to control the fine water generation unit and the air blow unit in such a manner that the fine water is supplied to the culture medium in the storage container by blowing air. Culturing can thus be started after a sufficient amount of fine water is supplied to the culture medium. This can facilitate culturing as water is more appropriately supplied to the material to be cultured from the beginning of the culturing. Various aspects of the culture method of the present disclosure described above may be used in this culture device, or a configuration implementing the steps of the culture method of the present disclosure may be added to this culture device.

In the culture device of the present disclosure, the storage container may include an opening and a container body containing the culture medium, and the control unit may perform, with the air passage being fitted in the opening, the water adsorption control and the water release control to supply the fine water toward the container body. The fine water can thus be more reliably supplied to the culture medium while reducing the entry of outside air into the container body (storage container).

The culture device of the present disclosure may further include a switching unit configured to selectively allow and block communication between the air passage and the storage container. The control unit may control the switching unit so as to block the communication between the air passage and the storage container in the water adsorption control, and may control the switching unit so as to allow the communication between the air passage and the storage container in the water release control. In this way, the water adsorption control and the water release control can be quickly switched, so that the fine water can be smoothly supplied to the culture medium.

In the culture device of the present disclosure, a lid that closes the opening may be attachable to the container body, and after the culture medium is inoculated with the material to be cultured, the lid may be attached to the container body and the storage container may be left to stand for a predetermined culture period without supply of the fine water. Culturing after inoculating the culture medium with the material to be cultured can thus be facilitated. The storage container may be left to stand at a location away from the fine water generation unit. After the fine water is supplied to the culture medium in the storage container, the storage container may be moved to the location away from the fine water generating unit. The storage container can thus be left to stand at a location suitable for culturing, and culturing can be facilitated.

### BRIEF DESCRIPTION OF THE DRAWINGS

[FIG. 1] FIG. 1 is a configuration diagram schematically showing the configuration of a culture device 10.
[FIG. 2] FIG. 2A is a configuration diagram schematically showing the configuration of a fine water generation cartridge 30, and FIG. 2B is a configuration diagram schematically showing the configuration of a fine water discharge element 34.
[FIG. 3] FIG. 3 is a process diagram showing an example of a culture method.
[FIG. 4] FIG. 4 is a flowchart showing an example of a fine water supply process.
[FIG. 5] FIG. 5 is an illustration showing the operation during water adsorption control.
[FIG. 6] FIG. 6 is a configuration diagram schematically showing the configuration of a culture device 100 according to a second embodiment.
[FIG. 7] FIG. 7 shows images showing the culture result of Example 1, where FIG. 7A is an image of a Petri dish taken from above, and FIG. 7B is an image of the Petri dish taken from below.
[FIG. 8] FIG. 8 shows images showing the culture result of Comparative Example 1, where FIG. 8A is an image of a Petri dish taken from above, and FIG. 8B is an image of the Petri dish taken from below.
[FIG. 9] FIG. 9 shows images showing the culture result of Comparative Example 2, where FIG. 9A is an image of a Petri dish taken from above, and FIG. 9B is an image of the Petri dish taken from below.
[FIG. 10] FIG. 10 shows images showing the culture result of Example 2, where FIG. 10A is an image of a Petri dish taken from above, and FIG. 10B is an image of the Petri dish taken from below.
[FIG. 11] FIG. 11 shows images showing the culture result of Comparative Example 3, where FIG. 11A is an image of a Petri dish taken from above, and FIG. 11B is an image of the Petri dish taken from below.
[FIG. 12] FIG. 12 shows images showing the culture result of Comparative Example 4, where FIG. 12A is an image of a Petri dish taken from above, and FIG. 12B is an image of the Petri dish taken from below.
[FIG. 13] FIG. 13 is an illustration showing an upper lid 52A attached to a container body 51.

### MODES FOR CARRYING OUT THE INVENTION

### [First Embodiment]

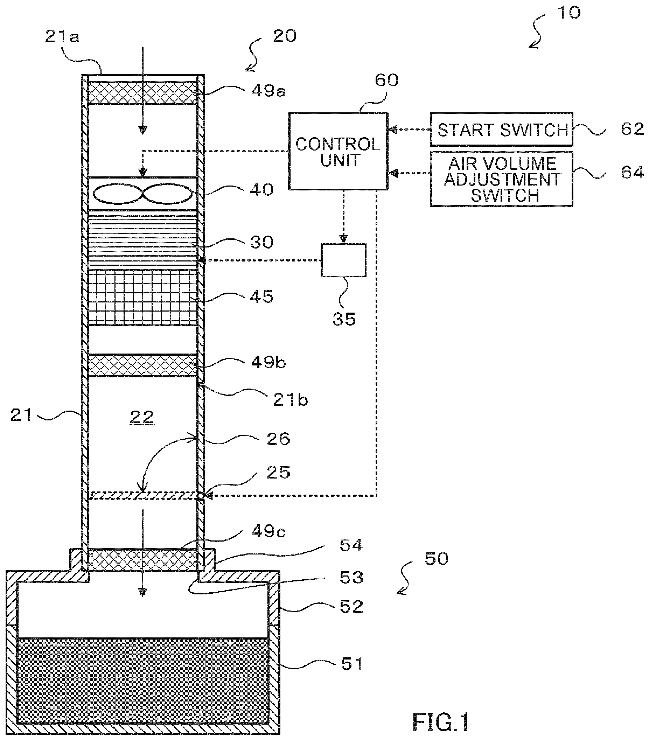

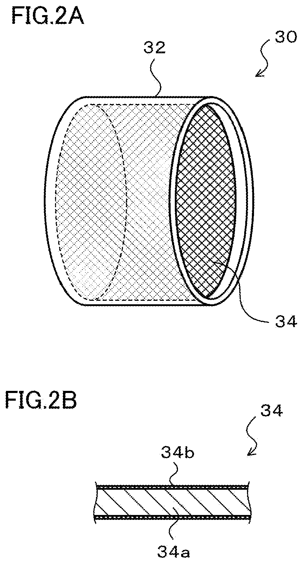

Next, a first embodiment of the present disclosure will be described with reference to the drawings. FIG. 1 is a configuration diagram schematically showing the configuration of a culture device 10 of the first embodiment, and FIG. 2 shows configuration diagrams schematically showing the configuration of a fine water generation cartridge 30. The culture device 10 includes a fine water supply unit 20 that supplies fine water, a storage container 50 that houses an object to be supplied with the fine water, and a control unit 60 that controls the entire device. In the present embodiment, the object to be supplied with the fine water is a culture medium to be used for culturing, and the culture medium before being inoculated with a material to be cultured is placed in the storage container 50 and is supplied with the fine water. Examples of the material to be cultured include microorganisms such as fungi like mold and yeast and bacteria, living cells, and tissue pieces. The culture medium may be any medium as long as it provides an appropriate culture environment for culturing the material to be cultured. Examples of the culture medium include, but are not limited to, agar media and grain media such as rice, barley, wheat, and potato.

The fine water supply unit 20 includes a duct 21 in which an air passage 22 is formed, the fine water generation cartridge 30, an energizing circuit 35, a fan 40, a temperature control cartridge 45, and filters 49a to 49c.

The duct 21 is a cylindrical member with both ends open, and forms a linear air passage 22. The duct 21 has an opening 21b that is open in a side wall so as to be perpendicular to the air passage 22, and is also provided with a switching unit 25 for switching opening and closing of the opening 21b.

The switching unit 25 includes a switching plate 26 that is operated by driving of a motor, not shown. When the switching plate 26 is in a normal position where the switching plate 26 forms part of an inner wall of the duct 21, the switching unit 25 closes the opening 21b so as not to allow air to flow through the opening 21b, and allows the air passage 22 to communicate with the storage container 50 (see solid lines in FIG. 1). When the switching plate 26 is operated to an operated position rotated 90 degrees from the normal position by driving of the motor, the switching unit 25 blocks communication between the air passage 22 and the storage container 50 and opens the opening 21b so as to allow air to pass through the opening 21b (see dashed lines in FIG. 1).

As shown in FIG. 2A, the fine water generation cartridge 30 includes a cylindrical case 32 having such an outside diameter that the case 32 can be placed in the air passage 22, and the fine water generation element 34 provided inside the case 32. As shown in FIG. 2B, the fine water generation element 34 includes a base material 34a and an electrically conductive polymer membrane 34b formed on the surface of the base material 34a.

The base material 34a is formed of an electrically conductive material such as a metal material like a stainless steel metal or a copper metal, a carbon material, or an electrically conductive ceramic material. Stainless steel metal foil containing aluminum is used in the present embodiment. The fine water generation element 34 is formed in the form of a corrugated plate, a honeycomb, a spiral, etc. so that air can flow therethrough and the surface area of the base material 34a (electrically conductive polymer membrane 34b) becomes as large as possible. The energizing circuit 35 including a power source and a switch is connected to the base material 34a. When the switch of the energizing circuit 35 is turned on by the control unit 60, the energizing circuit 35 is brought into an energizing state in which the energizing circuit 35 applies a current to the base material 34a. When the switch is turned off by the control unit 60, the energizing circuit 35 is brought into a non-energizing state in which current application to the base material 34a is stopped.

The electrically conductive polymer membrane 34b is formed of an electrically conductive polymer compound such as a thiophene-based electrically conductive polymer. In the present embodiment, the electrically conductive polymer membrane 34b is made of PEDOT/PSS (poly(3,4-ethylenedioxythiophene)/poly(styrene sulfonate)) among thiophene-based electrically conductive polymers. PEDOT/PSS has a core-shell structure having a core of PEDOT and a shell of sulfonic acid groups that are acidic functional groups that can hydrogen bond. The electrically conductive polymer membrane 34b has a stacked structure in which the shells of PEDOT/PSS are aligned, and forms, between the shells, nanochannels that are nanometer-sized channels such as two nanometers (nm). There are many sulfonic acid groups in the nanochannels. Therefore, when the amount of water on the surface of the electrically conductive polymer membrane 34b is large and the amount of water inside the electrically conductive polymer membrane 34b is small, the water that is present on the surface of the electrically conductive polymer membrane 34b moves into the electrically conductive polymer membrane 34b via the sulfonic acid groups inside the nanochannels due to the difference in concentration between the surface and inside of the electrically conductive polymer membrane 34b. The electrically conductive polymer membrane 34b thus adsorbs water. When the amount of water on the surface is small and the amount of water inside is large with water adsorbed inside, the water moves to the surface via the sulfonic acid groups inside the nanochannels due to the difference in concentration between the surface and the inside. Water is thus released from the electrically conductive polymer membrane 34b as fine water. When the temperature of the electrically conductive polymer membrane 34b is increased, quick release of water (fine water) is facilitated compared to the case where the water moves only due to the difference in concentration. When the temperature of the electrically conductive polymer membrane 34b is reduced, quick adsorption of water is facilitated compared to the case where the water moves only due to the difference in concentration. As described above, the fine water generation cartridge 30 (fine water generation element 34) changes to a water adsorbing state in which the electrically conductive polymer membrane 34b adsorbs water in air as the temperature decreases, and also changes to a water releasing state in which the adsorbed water is released from the electrically conductive polymer membrane 34b as the temperature increases. The thickness of the electrically conductive polymer membrane 34b can be determined as appropriate according to the required adsorption amount (release amount) of fine water. For example, when the electrically conductive polymer membrane 34b is formed with a thickness of 1 to 30 micrometers, it can adsorb the amount of water enough to release fine water in about a few seconds to several tens of seconds.

The fine water generation cartridge 30 releases non-charged fine water with a water particle size of 50 nanometers or less, for example, about 1 to 2 nanometers, from the electrically conductive polymer membrane 34b of the fine water generation element 34. The reason for this particle size is considered to be because the size of the nanochannels is 2 nanometers or less, and therefore a phenomenon occurs that water is forced out of the nanochannels due to an improved mobility of water and increase in pressure inside the nanochannels caused by an increase in temperature of the electrically conductive polymer membrane. Even if the water particles aggregate after being forced out, their particle size is distributed within a range of 50 nanometers or less. Generation of fine water by the fine water generation cartridge 30 (electrically conductive polymer membrane 34b) is described in detail in WO2020/054100, Japanese Unexamined Patent Application Publication No. 2019-018195 (JP 2019-018195 A), etc. filed by the applicant, and therefore will not be described in further detail.

The fan 40 blows air from an upper part to lower part of the duct 21 when rotationally driven in a first rotation direction. Therefore, when the switching plate 26 is in the normal position (see the solid lines in FIG. 1), air drawn into the air passage 22 from the opening 21a at the upper end of the duct 21 through the filter 49a can be sent to the storage container 50 through the filters 49b, 49c. The fan 40 blows air from the lower part to upper part of the duct 21 when rotationally driven in a second rotation direction opposite to the first rotation direction. Therefore, when the switching plate 26 is in the operated position (see the dashed lines in FIG. 1), air drawn into the air passage 22 from the opening 21b in the side wall of the duct 21 can be sent upward through the filter 49b. The fan 40 is rotationally driven by a motor, not shown, and is controlled by the control unit 60 by PWM (Pulse Width Modulation) control. The fan 40 may be a propeller fan, a Sirocco fan, etc.

The temperature control cartridge 45 is formed of a metal material and formed in the form of, for example, a corrugated plate, a honeycomb, a spiral, etc. so as to have a large heat capacity and high heat exchange efficiency. The temperature control cartridge 45 is disposed below the fine water generation cartridge 30. Therefore, when the fan 40 is rotationally driven in the first rotation direction, air that has passed through the fine water generation cartridge 30 into the temperature control cartridge 45 is cooled as it passes through the temperature control cartridge 45.

The storage container 50 includes a container body 51 that is open at the top and houses an object to be supplied with fine water, and an upper lid 52 that is attached to the top of the container body 51. The upper lid 52 includes an opening 53 formed at the center of its upper surface and having substantially the same inside diameter as the air passage 22, and a support portion 54 that stands upward from the edge of the opening 53 and supports the duct 21. Therefore, as shown in the figure, since the duct 21 is supported by the support portion 54, air containing fine water can be sent into the storage container 50 from above to fill the storage container 50 with the air containing fine water. Although not shown in the figure, an exhaust valve that exhausts air to the outside when the internal pressure of the storage container 50 increases is formed in the upper lid 52.

The control unit 60 is configured as a microprocessor having a CPU as a core component, and includes a ROM, a RAM, and input and output ports in addition to the CPU. An operation signal from a start switch 62 for starting operation of the culture device 10, an operation signal from an air volume adjustment switch 64 for adjusting the air volume of the fan 40, etc. are input to the control unit 60 via the input port. A drive signal for the motor that rotationally drives the fan 40, a drive signal for the switch of the energizing circuit 35, a drive signal for the motor of the switching unit 25, etc. are output from the control unit 60 through the output port.

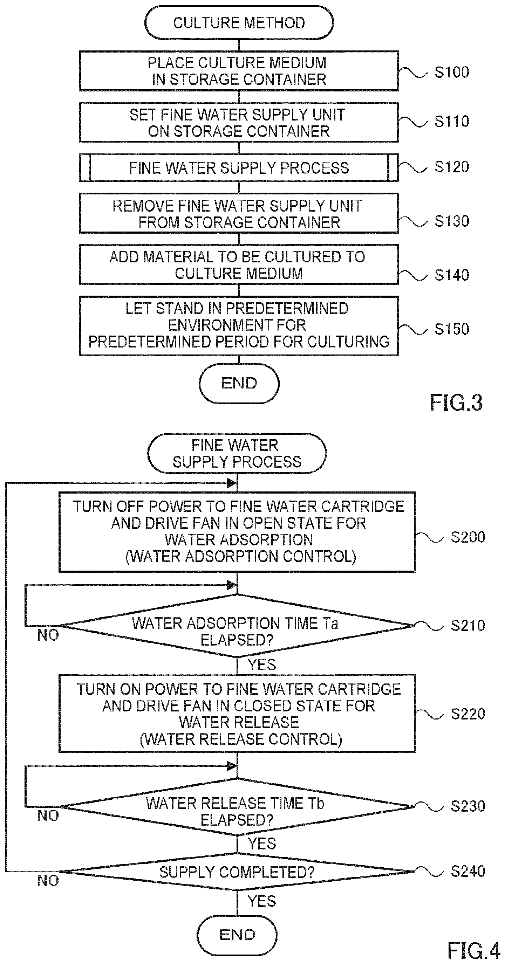

Next, a culture method using the culture device 10 configured as described above will be described. FIG. 3 is a process diagram showing an example of the culture method. An operator first places a culture medium in the container body 51 and attaches the upper lid 52 to the container body 51 (S100), and sets the fine water supply unit 20 (duct 21) on the upper lid 52 of the storage container 50 so that the culture device 10 becomes ready for operation (S110). Next, the operator operates the start switch 62 of the culture device 10 to perform a fine water supply process by the culture device 10 (S120). That is, in the present embodiment, the culture medium is placed in the storage container 50, and before the culture medium is inoculated with a material to be cultured, the process of supplying fine water to the culture medium is performed.

FIG. 4 is a flowchart showing an example of the fine water supply process. In the fine water supply process, the control unit 60 performs a water adsorption control in which the control unit 60 switches the duct 21 to an open state for water adsorption, turns off the power to the fine water generation cartridge 30, and drives the fan 40 to cause the electrically conductive polymer membrane 34b to adsorb water (S200), and waits for a predetermined water adsorption time Ta to elapse (S210).

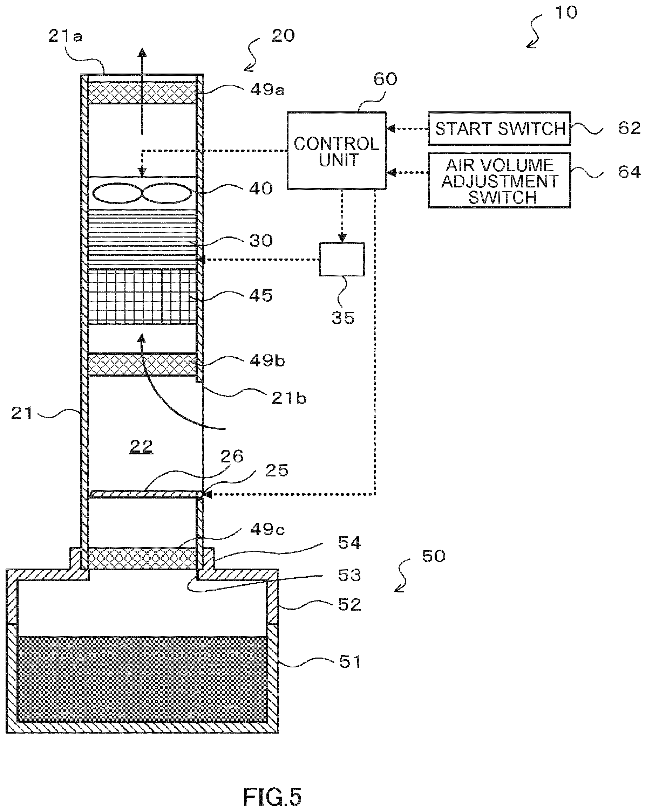

FIG. 5 is an illustration showing the operation during the water adsorption control. As shown in the figure, the switching unit 25 switches the switching plate 26 to the operated position to attain an open state in which communication between the air passage 22 and the storage container 50 is blocked and the opening 21b is opened. In this state, the fan 40 is rotationally driven in the second rotation direction. Therefore, air drawn into the air passage 22 from the opening 21b through the filter 49b flows toward the upper opening 21a (see arrows in FIG. 5). Since the control unit 60 turns off the energizing circuit 35, the energizing circuit 35 is brought into the non-energizing state in which current application to the base material 34a is stopped, so that the temperature of the electrically conductive polymer membrane 34b decreases and water adsorption is facilitated. As described above, in the water adsorption control in S200, the water adsorption by the fine water generation cartridge 30 is facilitated with the communication between the air passage 22 and the storage container 50 being blocked. Therefore, in the water adsorption control, it is possible to reduce the possibility of outside air flowing into the storage container 50.

When the control unit 60 determines in S110 that the water adsorption time Ta has elapsed, the control unit 60 switches the duct 21 to a closed state for water release, turns on the power to the fine water generation cartridge 30, and drives the fan 40 in the first rotation direction to perform a water release control (S220). The closed state for water release is the state shown by the solid lines in FIG. 1, and the fan 40 is rotationally driven in the first rotation direction. Therefore, air drawn into the air passage 22 from the opening 21a through the filter 49a passes through the filters 49b, 49c and flows toward the storage container 50 (see the arrows in FIG. 1). Since the control unit 60 turns on the energizing circuit 35, the energizing circuit 35 is brought into the energizing state in which the energizing circuit 35 applies a current to the base material 34a, so that the temperature of the electrically conductive polymer membrane 34b increases and release of fine water is facilitated. The released fine water is supplied into the storage container 50 by the blown air, and is directed to the culture medium. The fine water can thus be supplied while reducing the entry of outside air other than the air having passed through the fine water generation cartridge 30.

After performing the water release control in S220, the control unit 60 waits for a predetermined water release time Tb to elapse (S230). When the control unit 60 determines that the water release time Tb has elapsed, the control unit 60 determines whether supply of fine water is completed (S240). When the control unit 60 determines that the supply of fine water is not completed, the process returns to S200 to perform the water adsorption control. As described above, the control unit 60 alternately and repeatedly performs the water adsorption control and the water release control. The water adsorption time Ta and the water release time Tb can be set as appropriate according to the water adsorption capacity (water release capacity) of the fine water generation cartridge 30, the size of the storage container 50, the type of culture medium, etc. For example, the water adsorption time Ta is set to about twice the water release time Tb, and when the water release time Tb is set to 30 seconds or one minute, the water adsorption time Ta is set to one or two minutes, etc., although the present disclosure is not particularly limited to this.

For example, in S240, it may be determined that supply is completed when the processing time from the start of the fine water supply process reaches a predetermined amount of time such as a few hours or ten and several hours, or it may be determined that supply is completed when the number of times the water adsorption control and the water release control are repeated reaches a predetermined value. The predetermined amount of time for the fine water supply process can be determined as appropriate according to the type and amount of the target culture medium. However, in order to direct fine water to the entire culture medium to allow the culture medium to sufficiently absorb the fine water, the predetermined amount of time is preferably, for example, 12 hours or more, is more preferably 20 hours or more. A humidity sensor may be placed in the storage container 50, and whether supply is completed may be determined by referring to the detected humidity. When the control unit 60 determines in S240 that supply of fine water to the culture medium is completed, the control unit 60 ends the fine water supply process.

A grain culture medium that is a mixture of two or more types of grains is sometimes used as the culture medium. In that case, the culture medium may be mixed after the fine water supply process is performed, or the culture medium may be mixed before the fine water supply process is performed. For example, in the case of a grain culture medium that is a mixture of steamed soybeans and steamed wheat, the steamed soybeans subjected to the fine water supply process and supplied with fine water may be mixed with the steamed wheat subjected to the fine water supply process and supplied with fine water. Alternatively, the steamed soybeans and the steamed wheat may be mixed together and then subjected to the fine water supply process. That is, the culture medium placed in the container body 51 in S 100 may be either in a state where the culture medium can be inoculated with the material to be cultured as soon as fine water is supplied to the culture medium, or in a state in which a step such as mixing is necessary after fine water is supplied to the culture medium.

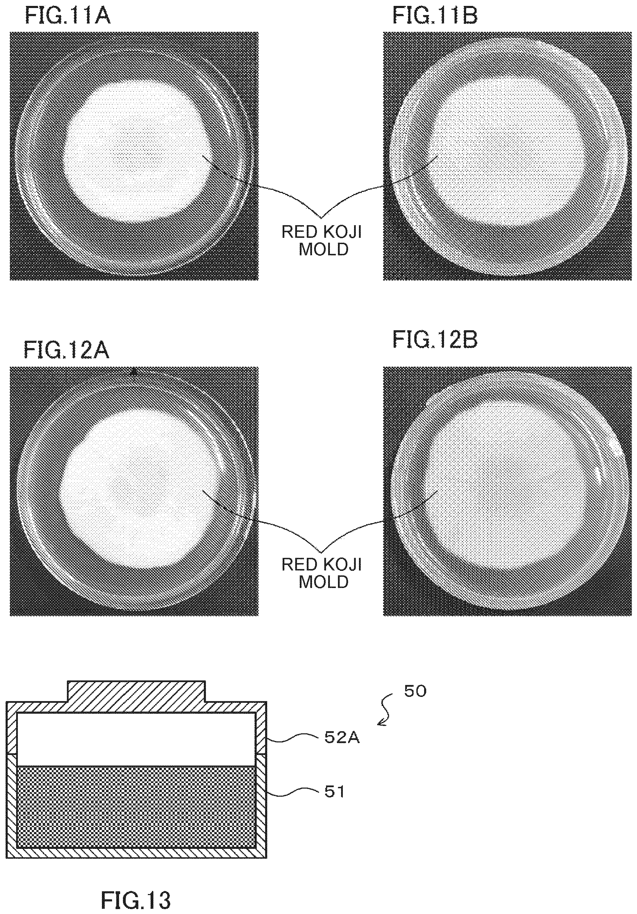

In the culture method of FIG. 3, after performing the fine water supply process in S120, the operator removes the fine water supply unit 20 from the storage container 50 (S130) and inoculates the culture medium with the material to be cultured (S 140). As described above, when any step is necessary before inoculating the cultured material with the material to be cultured, the culture medium may be inoculated with the material to be cultured in S140 after that step is performed. The operator then attaches an upper lid 52A that has no opening 53 for the duct 21 unlike the upper lid 52 to the container body 51 (see FIG. 13), and leaves the storage container 50 to stand for a predetermined culture period in a predetermined culture environment to culture the material to be cultured (S 150). The culture step thus ends. The predetermined culture environment is an environment in which the temperature, humidity, etc. are determined according to the type of material to be cultured, the type of culture medium, etc. The predetermined culture period is a period determined according to the type of material to be cultured, the type of culture medium, the size of the storage container 50, etc., and is, for example, a period of about several days.

In the culture method of the embodiment described above, after fine water is supplied from the fine water generation cartridge 30 (fine water generation element 34) to the culture medium before being inoculated with the material to be cultured, the culture medium is inoculated with the material to be cultured and is left to stand for culturing. Culturing is thus started after a sufficient amount of fine water is supplied to the culture medium. This can facilitate culturing as water is appropriately supplied from the culture medium to the material to be cultured from the beginning of the culturing. In the case where the culture medium is inoculated with a material to be cultured such as bacteria and the material is cultured while supplying fine water, the bacteria may be directly exposed to wind or the culture environment such as temperature may change significantly. Therefore, there is a risk that the bacteria may be weakened or killed as the bacteria is sensitive to the culture environment. In the present embodiment, after the culture medium is inoculated with the material to be cultured, the material is cultured by letting it stand. Therefore, such a risk can be prevented, and culturing can be appropriately facilitated.

Fine water with a particle size of 50 nanometers or less is supplied to the culture medium before being inoculated with the material to be cultured. Fine water with such a particle size is considered to be easily absorbed by the culture medium. Therefore, the culture medium is allowed to absorb a sufficient amount of water (fine water). Accordingly, water can be more appropriately supplied via the culture medium to the material to be cultured. Since the culture medium before being inoculated with the material to be cultured is allowed to absorb a sufficient amount of fine water, the fine water can remain in the culture medium for a long time and continue to supply fine water to the material to be cultured. The culturing can thus be more effectively facilitated.

Fine water is supplied into the storage container 50 while reducing the entry of outside air other than the air having passed through the fine water generation cartridge 30. Accordingly, the culture medium is less likely to be dried by the entry of the outside air, and the storage container 50 can be filled with air containing fine water. Therefore, supply of fine water to the culture medium can be facilitated.

After being inoculated with the material to be cultured, the culture medium is let stand for culturing with the lid of the storage container 50 closed. As a sufficient amount of fine water is supplied in advance to the culture medium, culturing can be facilitated by merely letting the culture medium stand after inoculating the culture medium with the material to be cultured.

In the first embodiment, the duct 21 of the fine water supply unit 20 is attached to the upper lid 52 (support portion 54) of the storage container 50, and fine water is supplied from directly above the culture medium. However, the present disclosure is not limited to this. For example, fine water may be supplied by any method such as attaching the duct 21 to the side of the storage container 50 or to the side of the upper lid 52 as long as the storage container 50 is filled with fine water. The storage container 50 is not limited to the container having the upper lid 52. The storage container 50 may be a container with an open top such as a Petri dish, and the duct 21 may be supported by a dedicated support portion so that the duct 21 is located above a Petri dish.

In the first embodiment, the duct 21 is directly attached to the upper lid 52 (support portion 54) of the storage container 50, so that fine water is supplied into the storage container 50 while reducing the entry of outside air other than the air having passed through the fine water generation cartridge 30. However, the present disclosure is not limited to this. For example, the storage container 50 may be a container with an open top, and the duct 21 may be supported by a dedicated support portion different from the upper lid 52, so that outside air may flow into the storage container 50. In that case, the duct 21 does not have the opening 21b, and the water adsorption control may simply be performed by merely changing the air blowing direction from the water release control.

In the first embodiment, fine water is continuously supplied (always supplied) by alternately performing the water adsorption control and the water release control in the fine water supply process. However, the present disclosure is not limited to this. For example, fine water may be intermittently supplied by allowing to set the operating time and the resting time and stopping the water adsorption control and the water release control during the resting time.

### [Second Embodiment]

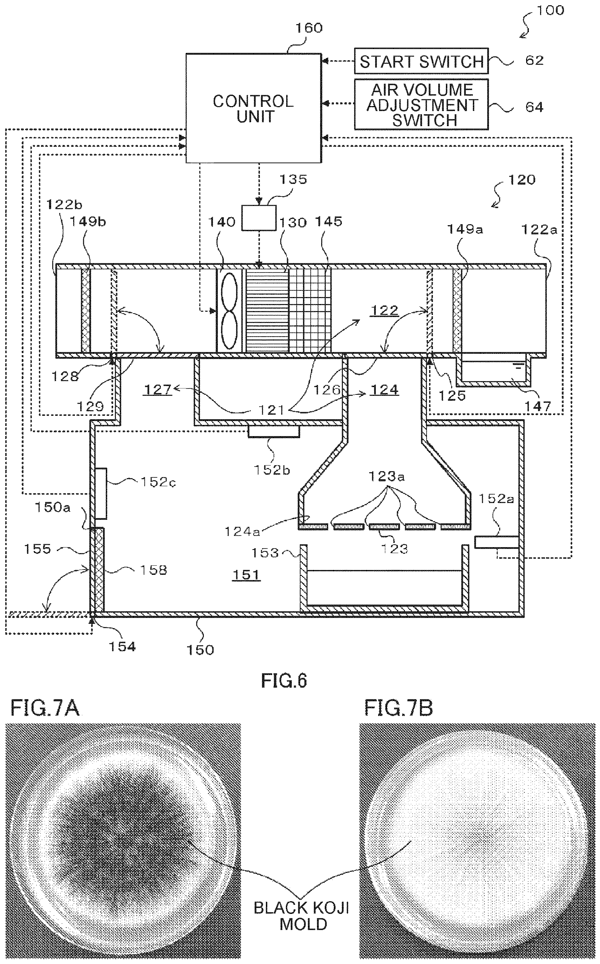

Next, a second embodiment of the present disclosure will be described with reference to the drawings. FIG. 6 is a configuration diagram schematically showing the configuration of a culture device 100 according to the second embodiment. The culture device 100 includes: a fine water supply unit 120 that supplies fine water; a culture case 150 where a container 153 such as a Petri dish for storing a material to be cultured and a culture medium is placed in a culture space 151; and a control unit 160 that controls the entire device. The fine water supply unit 120 includes an air passage 121, a fine water generation cartridge 130, an energizing circuit 135, a fan 140, and a temperature control cartridge 145. Since the components other than the air passage 121 have the same configuration as those in the first embodiment, description thereof will be omitted.

The air passage 121 includes a main passage 122, a first communication passage 124, and a second communication passage 127, and is provided with a first switching unit 125, a second switching unit 128, a water storage tank 147, and filters 149a, 149b. The main passage 122 is a tubular passage with both ends open. In the main passage 122, the water storage tank 147, the filter 149a, the temperature control cartridge 145, the fine water generation cartridge 130, the fan 140, and the filter 149b are provided in this order from a first opening 122a on one side toward a second opening 122b on the other side.

The first communication passage 124 and the second communication passage 127 are passages that allow communication between the main passage 122 and the culture case 150 (culture space 151). The first communication passage 124 is connected to the main passage 122 between the filter 149a and the temperature control cartridge 145, and extends into the culture space 151. The first communication passage 124 is formed such that the outlet 124a side in the culture space 151 has a larger inside diameter than the side connected to the main passage 122. The container 153 is disposed below the outlet 124a of the first communication passage 124 inside the culture space 151. The outlet 124a is formed in the form of a hood so as to cover the container 153, and a punching plate 123 having a plurality of through holes 123a is attached to the outlet 124a. The second communication passage 127 is connected to the main passage 122 between the filter 149b and the fan 140, and is also connected to the culture case 150. The component that is attached to the outlet 124a is not limited to the punching plate 123. A filter made of nonwoven fabric etc. may be attached to the outlet 124a. The punching plate 123, the filter, etc. may not be attached to the outlet 124a. Although the first communication passage 124 has a larger inside diameter on the outlet 124a side, the first communication passage 124 may have a constant inside diameter down to the outlet 124a side.

The first switching unit 125 includes a switching plate 126 that is operated by driving of a motor, not shown, and the second switching unit 128 includes a switching plate 129 that is operated by driving if a motor, not shown. When the switching plate 126 is in a normal position (initial position), the first switching unit 125 closes (blocks) the first communication passage 124, and opens the first opening 122a side so as to allow air to flow through the first opening 122a (see solid lines in FIG. 6). When the switching plate 126 is operated to an operated position rotated 90 degrees from the normal position by driving of the motor, the first switching unit 125 opens the first communication passage 124 and closes the first opening 122a side so as not to allow air to flow through the first opening 122a (see dashed lines in FIG. 6). When the switching plate 129 is in a normal position, the second switching unit 128 closes (blocks) the second communication passage 127, and opens the second opening 122b side so as to allow air to flow through the second opening 122b (see solid lines in FIG. 6). When the switching plate 129 is operated to an operated position rotated 90 degrees from the normal position by driving of the motor, the second switching unit 128 opens the second communication passage 127 and closes the second opening 122b side so as not to allow air to flow through the second opening 122b (see dashed lines in FIG. 6).

When the fan 140 is rotationally driven in the first rotation direction, the fan 140 blows air drawn into the main passage 122 from the first opening 122a through the filter 149a toward the second opening 122b. When the fan 140 is rotationally driven in the second rotation direction opposite to the first rotation direction, the fan 140 blows air drawn into the main passage 122 from the second opening 122b through the filter 149b toward the first opening 122a.

The water storage tank (water storage unit) 147 is formed between the first opening 122a and the filter 149a so that it can store water, and evaporated water is released into the main passage 122. When the fan 140 is rotationally driven in the first rotation direction, this evaporated water flows toward the fine water generation cartridge 130 through the filter 149a together with air drawn from the first opening 122a.

The culture case 150 includes: humidity sensors 152a to 152c that detect the humidity in the culture space 151; a communication port 150a formed in a part of a side wall of the culture case 150 and allowing communication between the inside and outside of the culture space 151; a switching unit (opening and closing unit) 154 that opens and closes the communication port 150a; and a filter 158 attached to the communication port 150a. The humidity sensor 152a detects the humidity near the outlet 124a of the first communication passage 124, that is, the humidity of air to be supplied to the container 153. The humidity sensor 152b detects the humidity in the upper part of the culture space 151. The humidity sensor 152c detects the humidity near the communication port 150a. The switching unit 154 includes a switching plate 155 that is operated by driving of a motor, not shown. When the switching plate 155 is in a normal position where the switching plate 155 forms part of the side wall of the culture case 150, the switching unit 154 closes the communication port 150a to block communication between the inside and outside of the culture space 151 (see solid lines in FIG. 6). When the switching plate 155 is operated to an operated position rotated 90 degrees to the outside from the normal position by driving of the motor, the switching unit 154 opens the communication port 150a and allows communication between the inside and outside of the culture space 151 (see dashed lines in FIG. 6).

The operation of the culture device 100 configured as described above will be described. The operation in the water adsorption control in S200 and the water release control in S220 in S120 (fine water supply process in FIG. 4) of the culture method of the first embodiment will be described.

In the water adsorption control, the control unit 160 performs a water adsorption control (normal water adsorption control) in which the control unit 160 switches the air passage 121 to a communicating state for water adsorption, turns off the power to the fine water generation cartridge 130, and drives the fan 140 at a predetermined air volume (first air volume) to cause the electrically conductive polymer membrane 34b to adsorb water. In the communicating state for water adsorption, the first switching unit 125 closes the first communication passage 124 and opens the first opening 122a side, the second switching unit 128 closes the second communication passage 127 and opens the second opening 122b side, and the fan 140 is rotationally driven in the first rotation direction. Therefore, air drawn into the main passage 122 through the first opening 122a flows toward the second opening 122b, so that water evaporated from the water storage tank 147 also flows toward the fine water generation cartridge 30 through the filter 149a. Since the energizing circuit 35 is turned off, the temperature of the electrically conductive polymer membrane 34b decreases, and water adsorption is facilitated.

In the water release control, the control unit 160 performs a water release control (normal water release control) in which the control unit 160 switches the air passage 121 to a communicating state for water release, turns on the power to the fine water generation cartridge 30, and drives the fan 40 at an air volume (second air volume) smaller than the predetermined air volume to supply fine water release from the electrically conductive polymer membrane 34b into the culture space 151. In the communicating state for water release, the first switching unit 125 opens the first communication passage 124 and closes the first opening 122a side, the second switching unit 128 opens the second communication passage 127 and closes the second opening 122b side. Therefore, the fan 140 is rotationally driven in the second rotation direction with air being allowed to flow between the first communication passage 124 and the second communication passage 127 in the main passage 122. Therefore, air blown from the fan 140 passes through the fine water generation cartridge 130 and the temperature control cartridge 145 in this order, flows into the culture space 151 from the first communication passage 124, and returns to the main passage 122 through the second communication passage 127. Since the energizing circuit 35 is turned on, the temperature of the electrically conductive polymer membrane 34b increases and release of fine water is facilitated. Therefore, the released fine water is supplied into the culture space 151 by the blown air and is directed to the culture medium in the container 153.

In the second embodiment, in addition to the normal water adsorption control and water release control, a humidity adjustment control is performed in which, when the humidity in the culture space 151 becomes higher than a predetermined humidity, the air passage 121 is switched to a communicating state for adjustment, and the power to the fine water generation cartridge 30 is turned off, and the fan 140 is driven at an air volume (second air volume) smaller than the predetermined air volume to circulate outside air in the culture space 151. In the communicating state for adjustment, the first switching unit 125 opens the first communication passage 124 and closes the first opening 122a side, the second switching unit 128 closes the second communication passage 127 and opens the second opening 122b side, the switching unit 154 opens the communication port 150a to allow communication between the inside and outside of the culture space 151, and the fan 140 is rotationally driven in the second rotation direction. Therefore, air (outside air) entering from the second opening 122b flows into the culture space 151 from the first communication passage 124 and flows to the outside from the communication port 150a. Since the power to the energizing circuit 35 is turned off, the temperature of the electrically conductive polymer membrane 34b decreases. Therefore, release of fine water is reduced. As described above, in the humidity adjustment control, outside air is introduced into the culture case 150 from the air passage 121 to make an adjustment to reduce the humidity in the culture space 151. The fan 140 may be configured so that the air volume can be set to different values for the water adsorption control, the water release control, and the humidity adjustment control by the air volume adjustment switch 64.

The humidity adjustment control of the second embodiment is not essential, and the humidity adjustment control may not be performed. In such a case, the culture case 150 does not have the communication port 150a, and the switching unit 154 may not be provided. When the humidity in the culture space 151 become higher than the predetermined humidity, the water release control may be stopped. In the case where the humidity tends to increase, such as frequently exceeding the predetermined humidity, an adjustment may be made such as reducing the water release time Tb. Although the culture case 150 is provided with the three humidity sensors 52a to 52c, the culture case 150 need only be provided with one or more humidity sensors. In the second embodiment, the water storage tank 147 is provided. However, the present disclosure is not limited to this, and the water storage tank 147 may not be provided. In the second embodiment, the temperature control cartridge 145 is provided in the main passage 122. However, the present disclosure is not limited to this, and the temperature control cartridge 145 may be provided in the first communication passage 124.

In the first embodiment and the second embodiment, the temperature control cartridges 45, 145 are provided. However, the present disclosure is not limited to this, and the temperature control cartridges 45, 145 may not be provided.

In the first embodiment and the second embodiment, when the culture medium supplied with fine water is inoculated with the material to be cultured and the material is cultured (S 140, S 150), the culturing is performed without supplying fine water. However, the present disclosure is not limited to this, and culturing may be performed while supplying fine water during a culture period after the culture medium is inoculated with the material to be cultured. For example, fine water may be supplied only during part of the culture period, such as at the beginning of the culture period. Although fine water is supplied directly from the fine water generation cartridge 30 (130), the present disclosure is not limited to this. Fine water may be supplied by any method as long as fine water having a particle size of 50 nanometers or less is supplied to the culture medium before being inoculated with the material to be cultured. For example, fine water may be supplied by leaving the culture medium to stand in an environment into which fine water is released.

In the first embodiment and the second embodiment, the water adsorption time Ta, the water release time Tb, and the rest time between the water adsorption control and the water release control may be adjusted, and the air volumes for the water adsorption control and the water release control may be adjusted, or whether supply of fine water is completed may be determined, all based on the humidity(ies) detected by the humidity sensor(s).

The correspondence between the main elements of the embodiments and the main elements of the present disclosure described in the section "Means for Solving the Problem" will be described. In the embodiments, S 100 of the culture method corresponds to step (a), S120 corresponds to step (b), and S140 and S150 correspond to step (c). The culture device 10 (100) corresponds to the "culture device," the air passage 22 (122) corresponds to the "air passage," the fan 40 (140) corresponds to the "air blow unit," the fine water generation cartridge 30 (130) corresponds to the "fine water generation unit," and the control unit 60 (160) corresponds to the "control unit." The storage container 50 (container 153) corresponds to the "storage container."

The correspondence between the main elements of the embodiments and the main elements of the present disclosure described in the section "Means for Solving the Problem" is an example for specifically illustrating the modes in which the embodiments carry out the present disclosure described in the section "Means for Solving the Problem." Therefore, this correspondence is not intended to limit the elements of the present disclosure described in the section "Means for Solving the Problem." In other words, the present disclosure described in the section "Means for Solving the Problem" should be interpreted based on the description in that section, and the embodiments are merely specific examples of the present disclosure described in the section "Means for Solving the Problem." Although the modes for carrying out the present disclosure are described above based on the embodiments, the present disclosure is not limited to these embodiments in any way, and may be carried out in various forms without departing from the spirit and scope of the present disclosure.

### Examples

Hereinafter, examples in which culturing was performed using the culture device 10 of the present disclosure will be described as examples. The present disclosure is not limited to Examples 1 and 2 below.

### (Supply of Fine Water)

In Example 1, a CP-added potato dextrose agar medium was placed in a Petri dish as the storage container 50, the Petri dish was set in the culture device 10, and the fine water supply process was performed for 21 hours to direct fine water to the medium. The process was performed in a clean bench at a temperature of 25°C and a humidity of 40%. The water adsorption control and the water release control were alternately and repeatedly performed with the above water adsorption time Ta of two minutes and the above water release time Tb of one minute.

### (Inoculation)

After the fine water supply process was performed for 21 hours, the Petri dish was removed from the culture device 10, and 20 µL of a suspension of black koji mold (NBRC4066) was dropped onto the CP-added potato dextrose agar medium that had been supplied with fine water. Black koji mold is not limited in its origin, and may be of any commonly available type.

### (Culturing)

Thereafter, a lid (upper lid with no opening) was attached to the Petri dish, and the Petri dish was left in a constant temperature bath at 30°C for six days.

As comparative examples, Comparative Examples 1 and 2 are shown in which, unlike the example, the fine water supply process was not performed on a CP-added potato dextrose agar medium before dropping the suspension. In Comparative Example 1, a CP-added potato dextrose agar medium was placed in a Petri dish, the lid was attached, the Petri dish was left to stand for 21 hours, and then 20 µL of a suspension of black koji mold (NBRC4066) was dropped. In Comparative Example 2, a CP-added potato dextrose agar medium was placed in a Petri dish, the Petri dish was left to stand in high humidity (80 to 90%RH) for 21 hours without attaching a lid to the Petri dish, namely with the Petri dish kept open, and then 20 µL of a suspension of black koji mold (NBRC4066) was dropped. In both Comparative Examples 1 and 2, after dropping the suspension of the black koji mold, the lid was attached to the Petri dish and the Petri dish was left to stand in a constant temperature bath at 30°C for six days as in the example.

### (Results)

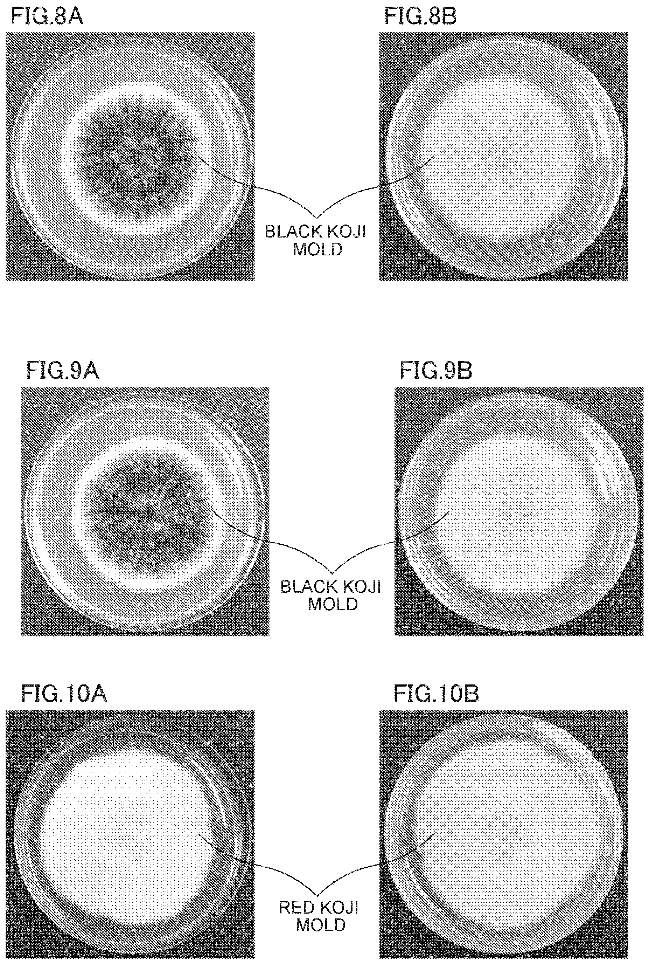

FIG. 7 shows images showing the culture result of Example 1. FIG. 8 shows images showing the culture result of Comparative Example 1. FIG. 9 shows images showing the culture result of Comparative Example 2. FIGS. 7 to 9 show images of the Petri dish taken from above (FIGS. 7A, 8A, and 9A) and images of the Petri dish taken from below (FIGS. 7B, 8B, and 9B) after being left to stand in the constant temperature bath for six days. In Comparative Examples 1 and 2, culturing did not spread over the entire surface of the Petri dish. In Example 1, however, the culturing spread over the entire surface of the Petri dish, showing superiority. In Example 1, more mycelial growth was observed in the outer periphery than in Comparative Examples 1 and 2. Since culturing was more facilitated in Example 1 than in Comparative Example 2 in which the culture medium was exposed to high humidity, it can be considered that Example 1 is more advantageous in that fine water that is water remains in the culture medium for a long time and continues to supply fine water to the black koji mold. The above results show that culturing of the black koji mold was facilitated by directing fine water to the culture medium before dropping the suspension of the black koji mold.

Next, Example 2 and Comparative Examples 3 and 4 will be described. In Example 2 and Comparative Examples 3 and 4, since the same steps were performed except that a suspension of red koji mold (NBRC4520) was dropped instead of black koji mold in Example 1 and Comparative Examples 1 and 2, description thereof will be omitted. Red koji mold is not limited in its origin, and may be of any commonly available type.

### (Results)

FIG. 10 shows images showing the culture result of Example 2. FIG. 11 shows images showing the culture result of Comparative Example 3. FIG. 12 shows images showing the culture result of Comparative Example 4. Like FIGS. 7 to 9, images of the Petit dish taken from above (FIGS. 10A, 11A, and 12A), and images of the Petit dish taken from below (FIGS. 10B, 11B, and 12B) are shown. In Comparative Examples 3 and 4, culturing did not spread over the entire surface of the Petri dish as in Comparative Example 1 and 2. In Example 2, on the other hand, as in Example 1, culturing spread over the entire surface of the Petri dish, showing superiority. In addition, more mycelial growth was observed in the outer periphery than in Comparative Examples 3 and 4. The above results show that culturing of the red koji mold was facilitated by directing fine water to the culture medium before dropping the suspension of the red koji mold. The result of the above Examples 1 and 2 show that, by supplying fine water to the culture medium before dropping a suspension of koji mold that is a material to be cultured, culturing is facilitated compared to Comparative Examples 1 and 3 in which no water is supplied to the culture medium and Comparative Examples 2 and 4 in which the culture medium was merely exposed to high humidity. In the case of exposing the culture medium to high humidity or steaming the culture medium, water is supplied in the form of water molecules. Therefore, water (water molecules) of about 0.38 nanometers is supplied. In Examples 1 and 2, on the other hand, water is supplied in the form of fine water. Therefore, water (fine water) with a lower limit of about one nanometer and an upper limit of 50 nanometers (1 to 50 nanometers) is supplied.

### INDUSTRIAL APPLICABILITY

The present disclosure is applicable in the field of the culture technology, the culture device manufacturing industry, etc.

## Claims

1. A culture method, comprising:
(a) a step of providing a culture medium;
(b) a step of supplying fine water from a fine water generation unit to the culture medium, the fine water generation unit being configured to change between a water adsorbing state in which an electrically conductive polymer membrane adsorbs water in air as a temperature decreases and a water releasing state in which the water adsorbed by the electrically conductive polymer membrane is released as fine water as the temperature increases; and
(c) a step of inoculating the culture medium supplied with the fine water with a material to be cultured and culturing the material.

2. A culture method, comprising:
(a) providing a culture medium;
(b) supplying fine water with a particle size of 50 nanometers or less to the culture medium; and
(c) a step of inoculating the culture medium supplied with the fine water with a material to be cultured and culturing the material.

3. The culture method according to claim 1 or 2, wherein in the (b), the fine water is also supplied to a storage container containing the culture medium while reducing entry of outside air other than air having passed through the fine water generation unit.

4. The culture method according to any one of claims 1 to 3, wherein in the (c), a storage container containing the culture medium is also left to stand in a closed state for a predetermined culture period without supplying the fine water.

5. A culture device that inoculates a culture medium with a material to be cultured and cultures the material, the culture device comprising:
an air passage communicating with a storage container;
an air blow unit disposed in the air passage;
a fine water generation unit that is disposed in the air passage and that changes between a water adsorbing state in which an electrically conductive polymer membrane adsorbs water in air as a temperature decreases and a water releasing state in which the water adsorbed by the electrically conductive polymer membrane is released as fine water as the temperature increases; and
a control unit that performs, with the culture medium before being inoculated with the material to be cultured being placed in the storage container, a water adsorption control in which the fine water generation unit is switched to the water adsorbing state and a water release control in which the fine water generation unit is switched to the water releasing state, and controls the fine water generation unit and the air blow unit in such a manner that the fine water is supplied to the culture medium in the storage container by blowing air.

6. The culture device according to claim 5, wherein:
the storage container includes an opening and a container body containing the culture medium; and
the control unit performs, with the air passage being fitted in the opening, the water adsorption control and the water release control to supply the fine water toward the container body.

7. The culture device according to claim 5 or 6, further comprising a switching unit configured to selectively allow and block communication between the air passage and the storage container, wherein the control unit controls the switching unit so as to block the communication between the air passage and the storage container in the water adsorption control, and controls the switching unit so as to allow the communication between the air passage and the storage container in the water release control.

8. The culture device according to claim 6, wherein a lid that closes the opening is attachable to the container body, and after the culture medium is inoculated with the material to be cultured, the lid is attached to the container body and the storage container is left to stand for a predetermined culture period without supply of the fine water.

9. The culture method according to claim 4, wherein the (c) is performed at a location away from a location where the (b) is performed.

10. The culture method according to claim 9, wherein the (c) further includes a step of, after the (b), moving the culture medium to the location away from the location where the (b) is performed.

11. The culture device according to claim 8, wherein the storage container is left to stand at a location away from the fine water generation unit.

12. The culture device according to claim 11, wherein after the fine water is supplied to the culture medium in the storage container, the storage container is moved to the location away from the fine water generating unit.

13. The culture method according to claim 1 or 2, wherein the material to be cultured is black koji

14. The culture device according to claim 5, wherein the material to be cultured is black koji.

15. The culture method according to claim 1 or 2, wherein the material to be cultured is red koji.

16. The culture device according to claim 5, wherein the material to be cultured is red koji.
